# EUROPEAN PATENT SPECIFICATION

(11) **EP 2 344 205 B1**
(45) Date of publication and mention of the grant of the patent: **24.05.2017**
(21) Application number: 09819752.8
(22) Date of filing: 06.10.2009
(51) Int. Cl.: A61K 49/00, A61P 9/10, A61B 5/05, A61B 5/0275, A61B 5/02

(54) **DETECTION OF ATHEROSCLEROSIS USING INDOCYANINE GREEN**
NACHWEIS VON ARTERIOSKLEROSE MITTELS INDOCYANINGRÜN
DÉTECTION DE L'ATHÉROSCLÉROSE UTILISANT DU VERT D'INDOCYANINE

(30) Priority: 10.10.2008 US 249715
(43) Date of publication of application: 20.07.2011
(73) Proprietor: The General Hospital Corporation, Boston, MA 02114 (US)
(72) Inventor: JAFFER, Farouc, A., Jamaica Plain MA 02130 (US)
(74) Representative: Rupprecht, Kay
(86) International application number: PCT/US2009/059691
(87) International publication number: WO 2010/042512

(56) References cited:
- WO-A1-01/22870
- WO-A2-03/057259
- US-A1- 2004 156 782
- US-A1- 2005 118 103
- ASHVIN N. PANDE ET AL: "Detection of macrophage activity in atherosclerosis in vivo using multichannel, high-resolution laser scanning fluorescence microscopy", JOURNAL OF BIOMEDICAL OPTICS, vol. 11, no. 2, 1 January 2006 (2006-01-01), page 021009, XP055102837, ISSN: 1083-3668, DOI: 10.1117/1.2186337
- JAFFER FAROUC A ET AL: "In vivo imaging of protease activity in atherosclerosis using a near-infrared fluorescence intravascular catheter", CIRCULATION, vol. 114, no. 18, Suppl. S, October 2006 (2006-10), page 414, XP002720554, & 79TH ANNUAL SCIENTIFIC SESSION OF THE AMERICAN-HEART-ASSOCIATION; CHICAGO, IL, USA; NOVEMBER 12 -15, 2006 ISSN: 0009-7322
- BANGHE ZHU ET AL: "Development of a near infrared fluorescence catheter: operating characteristics and feasibility for atherosclerotic plaque detection; Development of a near infrared fluorescence catheter", JOURNAL OF PHYSICS D: APPLIED PHYSICS, INSTITUTE OF PHYSICS PUBLISHING LTD, GB, vol. 38, no. 15, 7 August 2005 (2005-08-07), pages 2701-2707, XP020083256, ISSN: 0022-3727, DOI: 10.1088/0022-3727/38/15/024
- GARDNER C M ET AL: "Detection of Lipid Core Coronary Plaques in Autopsy Specimens With a Novel Catheter-Based Near-Infrared Spectroscopy System", JACC: CARDIOVASCULAR IMAGING, ELSEVIER, AMSTERDAM, NL, vol. 1, no. 5, 1 September 2008 (2008-09-01), pages 638-648, XP025430634, ISSN: 1936-878X, DOI: 10.1016/J.JCMG.2008.06.001 [retrieved on 2008-09-15]

## Description

### FILED OF THE INVENTION

The present invention relates generally to imaging of anatomical structures and, more particularly, to apparatus and process for the detection of atherosclerosis or atherosclerotic plaques. According to one exemplary embodiment of the present invention, indocyanine green can be used for the detection of atherosclerosis or atherosclerotic plaques using fluorescence and molecular imaging approaches.

### BACKGROUND INFORMATION

Despite advances in diagnosis and treatment, atherosclerotic vascular disease remains a significant cause of morbidity and mortality worldwide. As a result, there are currently significant efforts to detect high-risk, or vulnerable, atherosclerotic lesions prior to the onset of clinical events such as myocardial infarction or stroke.

The permeability of atherosclerotic plaques is a well-known phenomenon. The insudation of plasma proteins in atherosclerotic plaques is one of the earliest signs of atherogenesis. Immunohistochemical analysis of atherosclerotic specimens has revealed that increasing albumin insudation has a strong association with plaque growth.

This endothelial permeability is also correlated with plaque neovascularization and angiogenesis. Pathological neovascularization of atheromatous plaques may play a role in plaque destabilization, intraplaque hemorrhage, and ultimately, plaque vulnerability. It has been observed that neovascularization of plaques is associated with plaque rupture, higher-risk histological features such as a lipid-rich pool, and greater degrees of inflammation.

Current methods of MR imaging of large vessel atherosclerosis with contrast enhancement already exploit these phenomenon of permeability and neovascularization of plaques. Gadolinium and Gadolinium-based agents such as gadofluorine and MS-325 have been used to identify areas of microvessel growth or endothelial permeability. These agents presumably diffuse into plaque, either with or without binding plasma proteins such as albumin. However, current MRI approaches have limited ability to image smaller vessels such as human coronary arteries, due to limits on spatial resolution and sensitivity.

Fluorescent dyes, such as indocyanine green (ICG), have been used for years in connection with angiography to diagnose and treat vascular abnormalities that occur in the eye, e.g., choroidal neovascularization (CNV). ICG is an intravenous tricarbocyanine dye which has properties of near-infrared fluorescence and has been used in clinical medicine for many years. It is rapidly bound to plasma proteins (> 95% albumin, the remainder to alpha-globulins) and in blood achieves maximal absorbance at about 805 nm and emission at about 830 nm. It is likely non-toxic and currently used for retinal angiography, cardiac output measurement, and liver function assessment. ICG has a short half-life, but bound to proteins, it is used as an intravascular angiographic agent for about 40 minutes, and can be eliminated from the bloodstream in about 2 hours. ICG can be rapidly excreted unmetabolized into the bile, and possesses a very favorable safety profile with a less than about 0.1% sever side effect incidence.

Further, the medical uses of fluorescent dyes, such as ICG, outside of the foregoing diagnosis and treatment procedures has been relatively limited. ICG has also been utilized to provide fluorescent vascular angiograms during cardiac surgery, in particular with a clinical fluorescence reflectance imaging (FRI) system. Other known uses for ICG are limited to diagnostic procedures, such as determining cardiac output, hepatic function and liver blood flow.

Based on the correlations between plaque inflammation, neovascularization, and permeability, an NIRF agent would be a useful tool in the detection of biologically vulnerable plaques. In particular, it could potentially be useful as an agent for catheter-based detection, or as an adjunct to noninvasive MR-based detection with gadolinium agents. The specific use of ICG to detect vulnerable plaques in human coronary arteries, the cause of heart attacks, could be of significant clinical importance, as MRI has limited role in coronary imaging. Jaffer A Farouc et al., "In vivo imaging of protease activity in atherosclerosis using a near-infrared fluorescence intravascular catheter", Circulation, vol. 114, no. 18, Suppl. S, October 2006, page 414, discloses imaging proteolytic activity in atherosclerosis using a protease-activatable near-infrared fluorescence imaging agent.

Ashvin N Pande et al.: "Detection of macrophage activity in atherosclerosis in vivo using multichannel, high-resolution laser scanning fluorescence microscopy", Journal of biomedical optics, vol. 11, no. 2, March/April 2006, page 021009, discloses imaging macrophage activity in atherosclerosis using a macrophage-targeted, near-infrared fluorescent magnetofluorescent nanoparticle. While the ability of ICG to provide angiography of vessels and silhouettes of plaques may be apparent (but not yet demonstrated), the ability of ICG to specifically enhance atherosclerotic plaques with high-risk features (including neovessels, inflammation, and lipid) remains unknown.

### OBJECTS AND SUMMARY OF THE INVENTION

It is one exemplary object of the present invention to overcome certain deficiencies and shortcomings of the prior art systems and techniques (including those described herein above), and provide an exemplary system, process and method for the detection of atherosclerosis and atherosclerotic plaques using indocyanine green (ICG) in fluorescence and molecular imaging approaches.

According to one exemplary embodiment of the present invention, ICG for use in a method for imaging at least one portion of an anatomical structure is provided, the method comprising providing an indocyanine green (ICG) agent to the at least one portion, and determining whether at least one plaque structure associated with the at least one portion includes at least one of an angiogenesis or an inflammation based on the interaction between the ICG agent and the at least one portion. The anatomical structure can be a blood vessel. The angiogenesis can include at least one atherosclerosis characteristic. The inflammation can include at least one atherosclerosis characteristic. The lipid can include at least one atherosclerosis characteristic. The plaque cells can include at least atherosclerosis characteristic. The lack of extracellular matrix can include at least one atherosclerosis characteristic. The lack of a thick fibrous cap can include at least one atherosclerosis characteristic.

The method according to an exemplary embodiment can further comprise, prior to the determination, placing an arrangement to obtain information regarding the at least one portion in a vicinity of the at least one portion, wherein the information used to perform the determination. The placement can be provided intravascularly via catheters or angioscopes, or can be provided non-invasively. The arrangement can be a hand-held arrangement.

The determination can be performed after the ICG is agent provided to the at least one portion for a particular period of time. The determination is performed by measuring fluorescence of the at least one portion as effected by the ICG agent. The arrangement can be configured to quantify fluorescence of the at least one portion as effected by the ICG agent.

The arrangement can be further configured to image the at least one portion based on fluorescence of the at least one portion as effected by the ICG agent. The determination can include determining an efficacy of a particular drug based on the at least one of the inflammation or the angiogenesis or the lipid or the plaque cells, or the lack of extracellular matrix or the lack of a thick fibrous cap. The determination can include determining a particular therapy for at least one patient based on the at least one of the inflammation or the angiogenesis or the lipid or the plaque cells, or the lack of extracellular matrix or the lack of a thick fibrous cap.

According to another exemplary embodiment of the present invention, ICG for use in a method for imaging at least one portion of at least one blood vessel can be provide, the method comprising providing an indocyanine green (ICG) agent to the at least one portion, and determining whether the at least one portion includes at least one of an angiogenesis or an inflammation based on the interaction between the ICG agent and the at least one portion, wherein the at least one blood vessel is accessible via an intravascular arrangement. The blood vessel can be a coronary blood vessel, a carotid blood vessel, a cerebral blood vessel, an aorta blood vessel, an iliac blood vessel, a mesenteric blood vessel, a femoral blood vessel, a renal blood vessel, or other peripheral blood vessel.

According to another exemplary embodiment of the present invention, ICG for use in a method for imaging at least one portion of an anatomical structure is provided, the method comprising providing an indocyanine green (ICG) agent to the at least one portion which includes at least one stent, and determining whether a tissue surrounding the stent includes inflammation or angiogenesis or the lipid or the plaque cells, or the lack of extracellular matrix or the lack of a thick fibrous cap based on the interaction between the ICG agent and the at least one portion.

Also disclosed herein is an apparatus for obtaining information regarding at least one portion of a biological structure, the apparatus comprising at least one first arrangement configured to generate a first electro-magnetic radiation to be forwarded to the at least one portion and receive, from the at least one portion, a second radiation associated with the first electro-magnetic radiation, wherein the second radiation is associated with an indocyanine green (ICG) agent provided in the at least one portion, and a second arrangement which is configured to obtain information associated with the second radiation, and generate at least one image of the at least one portion as a function of the second radiation, wherein the at least one second arrangement is configured to determine whether at least one plaque structure associated with the at least one portion includes at least one of an angiogenesis or an inflammation or the lipid or the plaque cells, or the lack of extracellular matrix or the lack of a thick fibrous cap based on the interaction between the ICG agent and the at least one portion.

Also disclosed herein is an apparatus for obtaining information regarding at least one portion of at least one blood vessel, the apparatus comprising at least one first arrangement configured to generate a first electro-magnetic radiation to be forwarded to the at least one portion and receive, from the at least one portion, a second radiation associated with the first electro-magnetic radiation, wherein the second radiation is associated with an indocyanine green (ICG) agent provided in the at least one portion, and a second arrangement which is configured to obtain information associated with the second radiation, and generate at least one image of the at least one portion as a function of the second radiation, wherein the at least one second arrangement is configured to determine whether the at least one portion includes at least one of an angiogenesis or an inflammation or the lipid or the plaque cells, or the lack of extracellular matrix or the lack of a thick fibrous cap based on the interaction between the ICG agent and the at least one portion, wherein the at least one blood vessel is accessible via an intravascular arrangement.

Also disclosed herein is an apparatus for obtaining information regarding at least one portion of a biological structure, the apparatus comprising at least one first arrangement configured to generate a first electro-magnetic radiation to be forwarded to the at least one portion and receive, from the at least one portion, a second radiation associated with the first electro-magnetic radiation, wherein the second radiation is associated with an indocyanine green (ICG) agent provided in the at least one portion which includes at least one stent, and a second arrangement which is configured to obtain information associated with the second radiation, and generate at least one image of the at least one portion as a function of the second radiation, wherein the at least one second arrangement is configured to determine whether a tissue surrounding the stent includes inflammation based on the interaction between the ICG agent and the at least one portion.

These and other objects, features and advantages of the present invention will become apparent upon reading the following detailed description of embodiments of the invention, when taken in conjunction with the appended claims.

### BRIEF DESCRIPTION OF THE DRAWINGS

Further objects, features and advantages of the invention will become apparent from the following detailed description taken in conjunction with the accompanying figures showing illustrative embodiments of the invention, in which:
FIG. 1 shows a block diagram of an exemplary embodiment of a system according to the present invention;
FIG. 2 shows a flow diagram of an exemplary embodiment of a method of the present invention;
FIGS. 3(a)-3(c) show exemplary images of coronary-sized vessels in a living rabbit; and
FIGS. 4(a)-4(g) show exemplary images of a method of detecting atherosclerotic plaque in a rabbit blood vessel.

Throughout the figures, the same reference numerals and characters, unless otherwise stated, are used to denote like features, elements, components or portions of the illustrated embodiments. Moreover, while the present invention will now be described in detail with reference to the figures, it is done so in connection with the illustrative embodiments.

### DETAILED DESCRIPTION OF EXEMPLARY EMBODIMENT(S)

Indocyanine green (ICG) is a common retinal angiographic agent, with FDA approval for decades and possessing an excellent safety profile. In an exemplary embodiment of the present invention, ICG can enhance atherosclerotic plaques with good plaque-to-background ratios in vivo. While ICG angiography can outline plaque silhouettes, similar to contrast angiography with x-ray or CT or other such modalities, an exemplary embodiment of the present invention can facilitate a molecular imaging of plaque neovessels using ICG. This exemplary approach can facilitate the detection of specific biological features of atheromata, and can enhances the utility of ICG in atherosclerosis studies. For example, the molecular imaging of angiogenesis (plaque neovessels) and/or inflammation is increasingly recognized as a novel approach to detect high-risk plaques.

Exemplary embodiments of the present invention can utilize ICG to facilitate imaging of plaque neovessels and/or other high-risk features of plaques including inflammation and apoptosis or lipid or plaque cells, or the lack of extracellular matrix or the lack of a thick fibrous cap. Using in vivo, ex vivo, and microscopic fluorescence imaging, ICG can be retained in atheroma and is thus a plaque-specific contrast agent. This can be due to specific targeting of epitopes on neovessels. Another possibility includes the intrinsic hydrophobicity of ICG which may facilitate its deposition into regions of the plaque such as the hydrophobic lipid core. Another possibility includes plaque cells, or the lack of extracellular matrix or the lack of a thick fibrous cap, all of which may facilitate diffusion of ICG (free or albumin bound) into higher-risk atheroma.

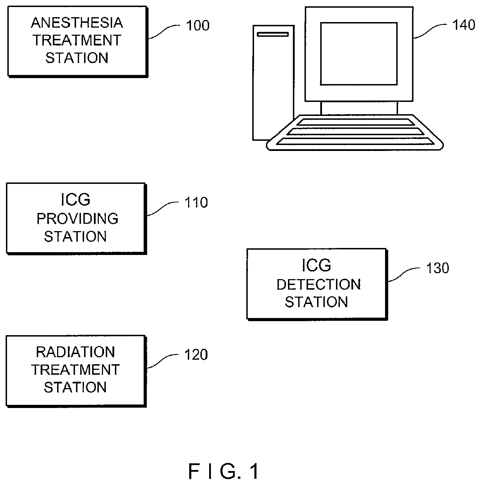

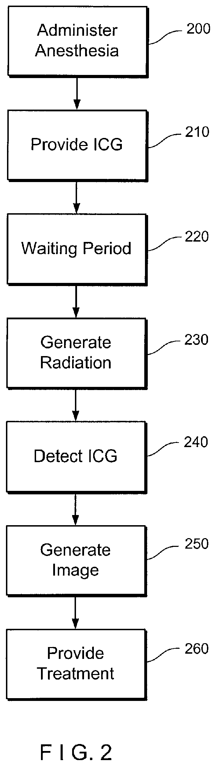

Block diagram and flow diagram of exemplary embodiments of the respective method and system of the present invention for detecting an atherosclerosis characteristic, such as angiogenesis, inflammation, a lipid, plaque cells, the lack of extracellular matrix, and/or the lack of a thick fibrous cap, are shown in FIGS. 1 and 2, respectively. As an initial matter, anesthesia can be administered to a patient in step 200 through an anesthesia treatment station 100. Then, ICG may be provided to a portion of an anatomical structure, such as a blood vessel, which could be a coronary blood vessel, a carotid blood vessel, a cerebral blood vessel, an aorta blood vessel, an iliac blood vessel, a mesenteric blood vessel, a femoral blood vessel, a renal blood vessel, or other peripheral blood vessel in step 210. The ICG providing station 110 can be a catheter or other similar arrangement, which can be provided intravascularly or non-invasively. A stent can be provided in the portion of the blood vessel during delivery of the ICG agent into the blood vessel.

For example, a period of time passes before the detection in step 220, which can preferably be between about 15-30 minutes, and can range from about a minute to 60 minutes. A first exemplary arrangement can be provided within a vicinity of the patient, which can comprise a radiation treatment station 120 and an ICG detection station 130. The radiation treatment station 120, which can be hand-held, may then be used to generate electro-magnetic radiation to the portion of the blood vessel at step 230. ICG can interact with the plaque, and the ICG detection station 130 may receive the electromagnetic radiation from the portion of the blood vessel where ICG is provided in step 240. A second exemplary arrangement, such as a computer having a display 140, can obtain information associated with the radiation from the ICG detection station 130, and may generate at least one image of the portion of the blood vessel as effected by the ICG agent in step 250.

ICG facilitates an emission of a fluorescence which can be measured by the first and second arrangement. The exemplary arrangements can be configured to quantify the fluorescence of the portion of the blood vessel as effected by the ICG agent. The second exemplary arrangement can determine whether a plaque structure associated the portion of the blood vessel includes an angiogenesis or an inflammation or the lipid or plaque cells, or the lack of extracellular matrix or the lack of a thick fibrous cap, based on the interaction of the blood vessel and the ICG agent. The display of the second exemplary arrangement can display the portion of the blood vessel and a fluorescent surrounding of the associated plaque in the portion of the blood vessel, if detected. The image can include the specific components that are identified using the technique according to the exemplary embodiment of the present invention.

Once a plaque structure has been located, a determination can be made regarding a particular drug or therapy for the patient based on the inflammation or angiogenesis if detected, in step 260.

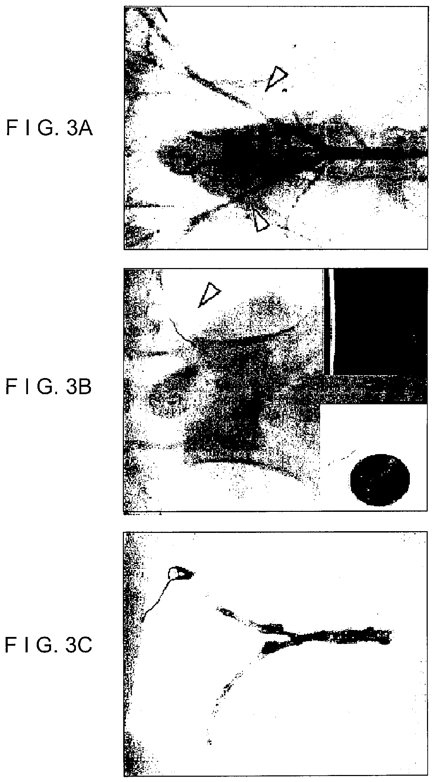

In Figs. 3(a)-3(c), a near-infrared fluorescence (NIRF) catheter protoype is percutaneously inserted into coronary-sized vessels in living rabbits. The catheter comprised a 0.36 mm/0.014 inch floppy radio-opaque tip with maximum outer diameter 0.48mm/0.019 inches. The focal spot for the 90-degree arc sensing catheter was 40±15 micrometer at a working distance of 2±1 mm. Angiography of balloon-injured, cholesterol-fed rabbits reveals visible lesions in the iliac arteries (arrowheads), as shown in Fig. 3(a). The NIRF catheter guidewire is easily delivered past ICG-enhanced stenoses in the iliac arteries (arrowhead), as seen in Fig. 3(b). Gross pathology reveals yellow-white atheromata in injured areas in the iliac arteries in Fig. 3(c).

### Experiment

An experiment demonstrating the use of ICG to detect atherosclerosis and atherosclerotic plaques in rabbits was performed as follows:

### Atherosclerosis

White rabbits (weight 3-3.5kg) underwent balloon-denudation of iliac arteries and hypercholsterolemic diet in order to develop inflamed atheromata. The rabbits were placed on a high-cholesterol diet (1% cholesterol and 5% peanut oil) for 1 week prior to balloon injury. After an overnight fast, anesthesia was induced with IM ketamine (35 mg/kg) and xylazine (5 mg/kg). Anesthesia was continued using inhaled isoflurane (1-5% v/v) and supplemental 02. Next a 3F Fogarty arterial embolectomy catheter was advanced into the common iliac vessels of the rabbits. The balloon was inflated to tension (0.6-1.0 ml). A total of 3 pullbacks were performed in the left and right iliac artery, as well as the infrarenal aorta. Following injury, the introducer was removed and the left carotid artery was ligated. Rabbits continued on the high-cholesterol diet for 4-8 weeks.

### Indocyanine Green

ICG was obtained from Sigma Molecular Formula: C₄₃H₄₇N₂Na0₆S₂, Molecular Weight: 775.0, CAS Number: 3599-32-4 Imax: 775 nm (water)3, Extinction Coefficient: EmM = 10.7-11.6 (393-394 nm), 7.788.82 (322 nm), 14.0-16.6 (262 nm), and 25.1-29.5 (220-223 nm). Other known names for Indocyanine green include 4,5-benzoindotricarbocyanine, Foxgreen, IC Green, ICG).

After an intravenous injection, the indocyanine green is bound to plasma protein, primarily albumin, and is rapidly taken up by the liver, and then excreted unchanged into the bile. For this reason, it is an indicator dye used for assessing cardiac output and liver function. Indocyanine is soluble in water (1 mg/ml). Indocyanine is not readily soluble in saline, and it should first be dissolved in water, then diluted with saline for applications requiring isotonic solutions.

### NIRF Catheter

The NIRF Catheter is a custom catheter-based sensing system that is compact and comprises a few components. For example, a continuous wave laser diode with an excitation wavelength of 750 nm served as an excitation source. The excitation light was filtered with a narrow band pass interference filter centered at 752 nm and with a 5 nm fullwidth-at-half-maximum (FWHM) in order to remove any residual laser scatter. Filtered excitation light was next guided using a multimode fiber after passing through a 3-dB beam splitter, and then coupled into a dedicated catheter prototype based on an optical coherence tomography wire. The catheter contains a 0.36 mm/0.014" floppy radio-opaque tip with outer diameter 0.41mm/0.016" housing a 62.5/125 micrometer multimode fiber of 200 cm length. At the end of the catheter, a prism then directed the light at 90 degrees with respect to the catheter and focused this light to a near diffraction-limited focal spot size of approximately 40±15 micrometer at a working distance of 2±1 mm.

### Catheter-based sensing of ICG enhancement of atherosclerotic plaques

Anesthesia was initiated as above, and a 5F introducer was placed into the right carotid artery and then delivered to the descending thoracic aorta under fluoroscopic guidance. IV heparin (150 units/kg) was next administered. A 5Fr balloon wedge catheter was placed through the sheath and the sheath was advanced to the abdominal aorta. An aortoiliac angiography was then performed. The NIRF catheter was then advanced through the balloon wedge catheter into the distal iliac arteries, distal to iliac atherosclerotic plaques. Manual pullbacks of the catheter over 20 seconds was performed in each iliac artery prior to and after ICG injection. The maximum voltage was recorded from each digitized pullback. The in vivo plaque target-to-background ratio (TBR) was thus calculated as TBR = (maximum voltage detected from all pullbacks) / (background voltage).

ICG was dissolved in sterile water (6 mg ICG in 1.2mL, concentration 5 mg/mL), and the entire volume was injected. Pullbacks were performed at baseline, and after injection, at the time points of 1 minute, 5 minutes, 10 minutes, 15 minutes, 20 minutes, 30 minutes, 45 minutes and 60 minutes. After completion of the experiment, the rabbits were perfused with saline and 4% paraformaldehyde at physiologic pressure.

### Ex Vivo Imaging

Resected atherosclerotic aortoiliac vessels underwent fluorescence reflectance imaging (FRI) with an excitation/emission filter 762nm/800nm and a 5 second exposure time. Light images were also obtained with an exposure time of 100 msec. Region-of-interest (ROI) analysis was performed using visual identification of plaques and normal background on FRI images. The plaque target-to-background ratio was defined as: TBR = (plaque ROI signal)/(adjacent vessel background ROI signal).

### Histopathology

After imaging, vessels were immersed in 4% paraformaldehyde for 24 hours. Vascular rings were then frozen in an optical cutting temperature compound for histopathological analysis. Serial 5 micrometer cryosections of rabbit atheromata were cut. Immunohistochemistry was performed using the avidin-biotin-peroxidase method. Briefly, sections treated with 0.3% of hydrogen peroxide were incubated for 60 minutes with primary or isotype control antibodies, followed by respective biotinylated secondary antibody. The reaction was visualized with aminoethylcarbazole substrate and counterstained with Harris hematoxylin solution. Immunoreactive cathepsin B, CD31 , and macrophages were identified using mouse monoclonal antibodies. Tissue sections were viewed using a microscope and images were captured using a digital camera.

### Exemplary Results

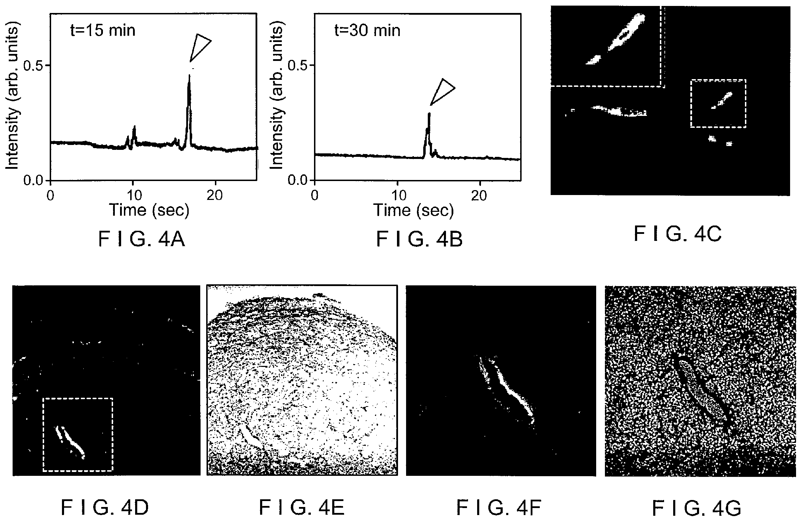

Initial voltage recordings from the intravascular space of the iliac arteries showed massive NIRF signal (>10V) immediately after injection, as anticipated with the injection of the fluorescent agent into the vasculature. Initially, no distinction was evident between atherosclerotic and normal vessel regions, due to a very high signal from ICG in the intravascular space. Delayed ("late-phase") catheter pullbacks revealed focal signal in the atherosclerotic plaques, as shown in FIGS. 4(a) and 4(b). FIG. 4(a) shows an exemplary graph illustrating a catheter pullback at 15 minutes after an ICG injection, showing a focal NIRF signal from ICG adjacent to an iliac artery plaque. FIG. 4(b) shows an exemplary graph illustrating a catheter pullback at 30 minutes showing detectable plaque ICG signal with a relatively lower SNR. High plaque-to-background ratios (>2:1) were present from 15-45 minutes post ICG injection.

These exemplary findings were confirmed by ex vivo fluorescence reflectance imaging, as shown in FIGS. 4(c)-4(g), providing very high plaque signal-to-background. In FIG. 4(c), an exemplary ex vivo NIRF image confirms a strong ICG signal in the atherosclerotic plaques in the iliac and aorta through a magnified inset. In FIG. 4(d), an image obtained using a fluorescence microscopy (x100) provides for a focal, perivascular ICG plaque signal (the dotted box) and adventitia. FIG. 4(e) illustrates an exemplary reference stained hemotoxylin and eosin image, x100. In FIG. 4(f), an exemplary image obtained using fluorescence microscopy (x200) of the dotted box in FIG. 4(d) indicates a strong ICG signal in a plaque neovessel. FIG. 4(g) shown an exemplary fusion brightfield ICG microscopy image. Correlative fluorescence microscopy demonstrated NIRF signal enhancement in plaque neovessels corroborating the in vivo and ex vivo imaging results.

Advantages of this exemplary embodiment of the system can include rapid signal enhancement, which likely facilitate clinical studies of the agent to identify high-risk plaques. Clinical studies can inject ICG at FDA-approved doses and detect ICG plaque enhancement within 15 minutes. Based on the microscopy and histology studies, ICG likely enhances high-risk plaques with plaque noevascularization and/or inflammation, or the lipid or plaque cells, or the lack of extracellular matrix or the lack of a thick fibrous cap, and not enhance low-risk plaques without these features. Another exemplary embodiment of the present invention includes a combined structural / molecular capability of ICG as the first-pass availability of ICG will allow initial fluorescence angiography of the coronary vessel, followed by late-phase ICG fluorescence molecular imaging of plaque angiogenesis/neovessels/inflammation. In addition to catheter based imaging, noninvasive imaging of other superificial arteries using this exemplary embodiment is also possible.

The exemplary embodiment of the method according to the present invention also fills yet unmet clinical need, e.g., the high-resolution detection of high-risk plaques in coronary sized vessels. Current molecular imaging approaches (e.g., MRI, nuclear, ultrasound, etc.) that attempt to detect plaque angiogenesis generally do not possess the combined sensitivity/target-to-background/resolution demands to image coronary sized plaques. The exemplary embodiment of the method according to the present invention provides an exemplary approach to accomplish this goal. The ready availability of ICG as an FDA-approved agent likely greatly facilitates its clinical use in vulnerable plaque detection and the ability to obtain a new indication to image atherosclerosis.

## Claims

1. Indiocyanine green (ICG) for use in a diagnosis method "in vivo" for an angiogenesis or inflammation, which involves imaging at least one plaque structure of an anatomical structure, comprising:
providing the ICG to the at least one plaque structure; and
determining whether the at least one plaque structure includes at least one of the angiogenesis and inflammation based on fluorescence caused by interaction between the ICG agent and the at least one plaque structure.

2. Indiocyanine green (ICG) for use according to claim 1, wherein the anatomical structure is at least one blood vessel.

3. Indiocyanine green (ICG) for use according to claim 1, wherein the angiogenesis includes at least one atherosclerosis characteristic.

4. Indiocyanine green (ICG) for use according to claim 1, wherein the inflammation includes at least one atherosclerosis characteristic.

5. Indiocyanine green (ICG) for use according to claim 1, wherein the diagnostic method further comprises:
prior to the determination, placing a device that is provided non-invasively to obtain information regarding the at least one plaque structure in a vicinity of the at least one plaque structure, wherein the information is used to perform the determination.

6. Indiocyanine green (ICG) for use according to claim 5, wherein the device is a hand-held device.

7. Indiocyanine green (ICG) for use according to claim 1, wherein the determination is performed at least one of (i) after the ICG is provided to the at least one plaque structure for a particular period of time, or (ii) by measuring fluorescence of the at least one plaque structure as affected by the ICG.

8. Indiocyanine green (ICG) for use according to claim 5, wherein the device is configured to at least one of (i) quantify fluorescence of the at least one portion as affected by the ICG, or (ii) image the at least one plaque structure based on fluorescence of the at least one plaque structure as affected by the ICG.

9. Indiocyanine green (ICG) for use according to claim 5, wherein the determination includes determining at least one of (i) an efficacy of a particular drug based on the at least one of the inflammation and the angiogenesis, or (ii) a particular therapy for at least one patient based on the at least one of the inflammation and the angiogenesis.

10. Indiocyanine green (ICG) for use in a diagnosis method "in vivo" for an angiogenesis or inflammation, which involves imaging at least one plaque structure of at least one blood vessel, comprising:
providing the ICG to the at least one plaque structure; and
determining whether the at least one plaque structure includes at least one of the angiogenesis and inflammation based on fluorescence caused by interaction between the ICG and the at least one plaque structure, wherein the at least one blood vessel is accessible via an intravascular device.

11. Indiocyanine green (ICG) for use according to claim 10, wherein the at least one blood vessel is at least one of a coronary blood vessel, a carotid blood vessel, a cerebral blood vessel, an aorta blood vessel, an iliac blood vessel, a mesenteric blood vessel, a femoral blood vessel, a renal blood vessel, or peripheral blood vessel.

12. Indiocyanine green (ICG) for use in a diagnosis method "in vivo" for an angiogenesis or inflammation, which involves imaging at least one plaque structure of an anatomical structure, comprising:
providing the ICG to the at least one plaque structure which includes at least one stent; and
determining whether a tissue surrounding the stent includes inflammation based on fluorescence caused by interaction between the ICG and the at least one plaque structure.

13. A method for imaging at least one plaque structure of an anatomical structure, comprising:
providing ICG to the at least one ex-vivo plaque structure; and
determining whether the at least one plaque structure includes at least one of an angiogenesis and inflammation based on fluorescence caused by interaction between the ICG and the at least one plaque structure.

## Patentansprüche

1. Indocyaningrün (ICG) zur Verwendung bei einem in vivo-Verfahren zur Diagnose einer Angiogenese oder einer Entzündung, wobei das Verfahren die Abbildung mindestens einer Plaquestruktur einer anatomischen Struktur beinhaltet, umfassend:
die Bereitstellung des ICG an der mindestens einen Plaquestruktur; und
die Bestimmung, ob die mindestens eine Plaquestruktur mindestens einen der Befunde Angiogenese und Entzündung aufweist, auf der Grundlage der durch die Wechselwirkung zwischen dem ICG-Agens und der mindestens einen Plaquestruktur hervorgerufen wird.

2. Indocyaningrün (ICG) zur Verwendung nach Anspruch 1, wobei es sich bei der anatomischen Struktur um mindestens ein Blutgefäß handelt.

3. Indocyaningrün (ICG) zur Verwendung nach Anspruch 1, wobei die Angiogenese mindestens ein Atherosklerose-Merkmal umfasst.

4. Indocyaningrün (ICG) zur Verwendung nach Anspruch 1, wobei die Entzündung mindestens ein Atherosklerose-Merkmal umfasst.

5. Indocyaningrün (ICG) zur Verwendung nach Anspruch 1, wobei das Diagnoseverfahren ferner Folgendes umfasst:
vor der Bestimmung das nicht-invasive Anbringen einer Vorrichtung, die zur Gewinnung von Informationen bezüglich der mindestens einen Plaquestruktur bereitgestellt wird, in der Nähe der mindestens einen Plaquestruktur, wobei die Informationen für die Bestimmung verwendet werden.

6. Indocyaningrün (ICG) zur Verwendung nach Anspruch 5, wobei es sich bei der Vorrichtung um eine handgeführte Vorrichtung handelt.

7. Indocyaningrün (ICG) zur Verwendung nach Anspruch 1, wobei die Bestimmung (i) nach Bereitstellung des ICG an der mindestens einen Plaquestruktur für eine bestimmte Zeitspanne und/oder (ii) durch Messen der Fluoreszenz an der mindestens einen Plaquestruktur entsprechend der Einwirkung durch das ICG vorgenommen wird.

8. Indocyaningrün (ICG) zur Verwendung nach Anspruch 5, wobei die Vorrichtung so aufgebaut ist, dass (i) die Fluoreszenz des mindestens einen Bereichs entsprechend der Einwirkung durch das ICG quantitativ bestimmt wird und/oder (ii) die mindestens eine Plaquestruktur auf der Grundlage der Fluoreszenz der mindestens einen Plaquestruktur entsprechend der Einwirkung durch das ICG abgebildet wird.

9. Indocyaningrün (ICG) zur Verwendung nach Anspruch 5, wobei die Bestimmung (i) das Feststellen der Wirksamkeit eines speziellen Arzneistoffs auf der Grundlage mindestens einer der Befunde Entzündung und Angiogenese und/oder (ii) das Festlegen einer speziellen Therapie für mindestens einen Patienten auf der Grundlage mindestens einer der Befunde Entzündung und Angiogenese umfasst.

10. Indocyaningrün (ICG) zur Verwendung bei einem in vivo-Verfahren zur Diagnose einer Angiogenese oder einer Entzündung, wobei das Verfahren die Abbildung mindestens einer Plaquestruktur mindestens eines Blutgefäßes beinhaltet, umfassend:
die Bereitstellung des ICG an der mindestens einen Plaquestruktur; und
die Bestimmung, ob die mindestens eine Plaquestruktur mindestens einen der Befunde Angiogenese und Entzündung aufweist, auf der Grundlage der durch die Wechselwirkung zwischen dem ICG und der mindestens einen Plaquestruktur hervorgerufenen Wechselwirkung, wobei das mindestens eine Blutgefäß über eine intravaskuläre Vorrichtung zugänglich ist.

11. Indocyaningrün (ICG) zur Verwendung nach Anspruch 10, wobei es sich bei dem mindestens einen Blutgefäß um ein koronares Blutgefäß, ein Karotis-Blutgefäß, ein zerebrales Blutgefäß, ein Aorta-Blutgefäß, ein Darmbein-Blutgefäß, ein mesenteriales Blutgefäß, ein femorales Blutgefäß, ein renales Blutgefäß und/oder ein peripheres Blutgefäß handelt.

12. Indocyaningrün (ICG) zur Verwendung bei einem in vivo-Verfahren zur Diagnose einer Angiogenese oder einer Entzündung, wobei das Verfahren die Abbildung mindestens einer Plaquestruktur einer anatomischen Struktur beinhaltet, umfassend:
die Bereitstellung des ICG an der mindestens einen Plaquestruktur, die mindestens einen Stent umfasst; und
die Bestimmung, ob ein das den Stent umgebende Gewebe eine Entzündung aufweist, auf der Grundlage der durch die Wechselwirkung zwischen dem ICG und der mindestens einen Plaquestruktur hervorgerufenen Fluoreszenz.

13. Verfahren zur Abbildung mindestens einer Plaquestruktur einer anatomischen Struktur, umfassend:
die Bereitstellung von ICG an mindestens einer ex vivo-Plaquestruktur; und
die Bestimmung, ob die mindestens eine Plaquestruktur mindestens einen der Befunde Angiogenese und Entzündung aufweist, auf der Grundlage der durch die Wechselwirkung zwischen dem ICG und der mindestens einen Plaquestruktur hervorgerufenen Fluoreszenz.

## Revendications

1. Vert d'indocyanine (ICG) pour utilisation dans une méthode de diagnostic "in vivo" pour une angiogenèse ou inflammation, qui implique l'imagerie d'au moins une structure de plaque d'une structure anatomique, comprenant :
pourvoir l'ICG à au moins une structure de plaque ; et
déterminer si au moins une structure de plaque comprend au moins l'une d'entre l'angiogenèse ou l'inflammation basées sur la fluorescence causée par l'interaction entre l'agent ICG et au moins une structure de plaque.

2. Vert d'indocyanine (ICG) pour utilisation selon la revendication 1, où la structure anatomique est au moins un vaisseau sanguin.

3. Vert d'indocyanine (ICG) pour utilisation selon la revendication 1, où l'angiogenèse comprend au moins une caractéristique d'athérosclérose.

4. Vert d'indocyanine (ICG) pour utilisation selon la revendication 1, où l'inflammation comprend au moins une caractéristique d'athérosclérose.

5. Vert d'indocyanine (ICG) pour utilisation selon la revendication 1, où la méthode de diagnostic comprend de plus :
avant de détermination, placer un dispositif non invasif pour obtenir l'information regardant au moins une structure de plaque dans un voisinage d'au moins une structure de plaque, où l'information est utilisée pour réaliser la détermination.

6. Vert d'indocyanine (ICG) pour utilisation selon la revendication 5, où le dispositif est un dispositif portatif.

7. Vert d'indocyanine (ICG) pour utilisation selon la revendication 1, où la détermination est réalisée au moins un des (i) après que l'ICG est pourvu au moins à une structure de plaque pour une période particulière de temps, ou (ii) après qu'en mesurant la fluorescence d'au moins une structure de plaque comme affectée par l'ICG.

8. Vert d'indocyanine (ICG) pour utilisation selon la revendication 5, où le dispositif est configuré au moins à un des (i) évaluer la quantité de fluorescence d'au moins une portion comme affectée par l'ICG, ou (ii) imaginer au moins une structure de plaque basée sur la fluorescence d'au moins une structure de plaque comme affectée par l'ICG.

9. Vert d'indocyanine (ICG) pour utilisation selon la revendication 5, où la détermination comprend déterminer au moins un des (i) une efficacité d'un médicament particulier basée sur au moins l'une d'entre l'inflammation et l'angiogenèse, ou (ii) une thérapie particulière pour au moins un patient basée sur au moins l'une d'entre l'inflammation et l'angiogenèse.

10. Vert d'indocyanine (ICG) pour utilisation dans une méthode de diagnostic "in vivo" pour une angiogenèse ou inflammation, qui implique l'imagerie au moins d'une structure de plaque d'au moins un vaisseau sanguin, comprenant :
pourvoir l'ICG au moins à une structure de plaque ; et
déterminer si au moins "une structure de plaque comprend au moins l'une d'entre l'angiogenèse ou l'inflammation basées sur la fluorescence causée par l'interaction entre l'ICG et au moins une structure de plaque, où au moins un vaisseau sanguin est accessible par un dispositif intravasculaire.

11. Vert d'indocyanine (ICG) pour utilisation selon la revendication 10, où au moins un vaisseau sanguin est au moins l'un d'entre un vaisseau sanguin coronaire, un vaisseau sanguin carotidien, un vaisseau sanguin cérébral, un vaisseau sanguin aortique, un vaisseau sanguin iliaque, un vaisseau sanguin mésentérique, un vaisseau sanguin fémoral, un vaisseau sanguin rénal, ou vaisseau sanguin périphérique.

12. Vert d'indocyanine (ICG) pour utilisation dans une méthode de diagnostic "in vivo" pour une angiogenèse ou inflammation, qui implique l'imagerie au moins d'une structure de plaque d'une structure anatomique, comprenant :
pourvoir l'ICG au moins à une structure de plaque qui comprend au moins un stent; et
déterminer si au moins un tissu environnant le stent comprend l'inflammation basée sur la fluorescence causée par l'interaction entre l'ICG et au moins une structure de plaque.

13. Méthode pour imaginer au moins une structure de plaque d'une structure anatomique, comprenant :
pourvoir l'ICG au moins à une structure de plaque ex-vivo; et
déterminer si au moins une structure de plaque comprend au moins l'une d'entre l'angiogenèse et l'inflammation basées sur la fluorescence causée par l'interaction entre l'ICG et au moins une structure de plaque.
